# EUROPÄISCHE PATENTANMELDUNG

(11) **EP 2 468 962 A1**
(43) Veröffentlichungstag der Anmeldung: **27.06.2012**
(21) Anmeldenummer: 11194212.4
(22) Anmeldetag: 19.12.2011
(51) Int. Cl.: E02D 29/12

(54) **Schachtbauwerk**

(30) Priorität: 21.12.2010 DE 202010016863 U
(71) Anmelder: Rehau AG & Co, 95111 Rehau (DE)
(72) Erfinder: Brückner, Pascal, 94244 Teisnach (DE); Ulbrich, Steffen, 96123 Litzendorf (DE); Meyer, Michael, 85072 Eichstätt (DE)

(57) **Zusammenfassung**

Die vorliegende Erfindung betrifft ein Schachtbauwerk (1) mit einer Einrichtung zur Abtrennung von Kondensaten eines Gases, insbesondere eines Biogases, mit einem Schachtboden (4), optional wenigstens einem Schachtmittelteil (3), optional einem Schachtkonus (2), wenigstens einer zugeordneten Gaszuführleitung (5), wenigstens einer zugeordneten Gasabführleitung (6), wenigstens einer Kondensatleitung (13) und wenigstens einer Vorrichtung zum Abscheiden und Auffangen von Kondensat aus Gaszuführ- und / oder Gasabführleitung (5, 6).

## Beschreibung

Die Erfindung betrifft ein Schachtbauwerk mit einer Einrichtung zur Abtrennung von Kondensaten eines Gases. Das Gas kann insbesondere ein Biogas sein.

Weiterhin betrifft die Erfindung auch eine Gasgewinnungsvorrichtung mit einem Schachtbauwerk.

Im Rahmen der Erschließung regenerativer Energien erlangt die Gewinnung von Biogas durch bakteriellen Abbau organischen Materials zunehmend an Bedeutung.

Dieses beim Abbau entstehende Biogas enthält herstellungsbedingt große Mengen an Wasserdampf sowie wasserlösliche, insbesondere auch gegenüber verschiedenen Stoffen aggressive Bestandteile.

Zur Verstromung des Biogases wird dieses aus dem Herstellungsbehältnis mittels Rohrleitungen zum Verstromungsaggregat geleitet. In den Rohrleitungen kondensiert dabei der Wasserdampf zumindest teilweise zusammen mit wasserlöslichen Bestandteilen aus und ist abzuführen.

Dadurch können die Verstromungsaggregate wirksam vor dem Einfluss von Feuchtigkeit und aggressiven Bestandteilen geschützt werden.

Nach dem Stand der Technik werden dazu verschiedene Gaskondensatabscheidersysteme aus Edelstahl- oder Betonschachtbauwerken genutzt.

Derartige Lösungen sind entweder zu aufwendig und teuer oder aber nicht dauerhaft genug gegenüber den aggressiven Bestandteilen, die in diesen Kondensaten enthalten sind.

Aufgabe der vorliegenden Erfindung ist es daher, ein Schachtbauwerk bereitzustellen, das in einfacher Weise modular aufbaubar ist, sich leicht installieren lässt, chemischen Angriffen der aggressiven Bestandteile der Kondensate dauerhaft widersteht, sowie die einschlägigen technischen Vorschriften, insbesondere, was das Gefahrenpotenzial angeht, erfüllt. Hier setzt die Erfindung ein, die darin besteht, ein Schachtbauwerk gemäß Anspruch 1 zur Verfügung zu stellen, welches eine Einrichtung zur Abtrennung von Kondensaten eines Gases, insbesondere eines Biogases aufweist.

Das Schachtbauwerk umfasst einem Schachtboden, optional wenigstens einem Schachtmittelteil, optional einem Schachtkonus, wenigstens einer zugeordneten Gaszuführleitung, wenigstens einer zugeordneten Gasabführleitung und wenigstens einer Kondensatleitung und wenigstens einer Vorrichtung zum Abscheiden und Auffangen von Kondensat aus Gaszuführ- und/oder Gasabführleitung.

Es hat sich hierbei als besonders günstig herausgestellt, wenn der Schachtboden, das optional wenigstens eine Schachtmittelteil, der optional eine Schachtkonus, die wenigstens eine zugeordnete Gaszuführleitung, die wenigstens eine zugeordnete Gasabführleitung und die wenigstens eine Kondensatleitung und die wenigstens eine Vorrichtung zum Abscheiden und Auffangen von Kondensat wenigstens anteilig aus Polymermaterial bestehen.

Hierdurch ist gewährleistet, dass das Schachtbauwerk als solches in einfacher Art und Weise modular je nach den Erfordernissen und örtlichen Gegebenheiten leicht zusammensetzbar ist und durch eine entsprechende Auswahl des Polymermaterials dauerhaft den im Kondensat vorhandenen aggressiven Bestandteilen widersteht.

Erfindungsgemäß kann hierbei insbesondere auf aus der Abwassertechnik bekannten Schachtbauteile, wie beispielsweise einem Schachtboden aus Polypropylen, einem Schachtmittelteil in variabler Höhe aus Polypropylen, einem Schachtkonus aus Polypropylen und Leitungen aus Polyethylen, die als Gaszuführleitung bzw. Gasabführleitung ausgebildet sind, zurückgegriffen werden.

Diese Bauteile lassen sich in einfacher Art und Weise verbinden, indem sie beispielsweise unter Nutzung von Dichtungen zusammengesteckt werden oder miteinander verschweißt werden.

Insbesondere beim Verschweißen der Bauteile lassen sich gasdichte Verbindungen herstellen, die im Hinblick auf die Sicherheit eines derartigen Schachtbauwerks von entsprechender Bedeutung sind.

Die einzelnen Bauteile des Schachtbauwerks, wie beispielsweise der Schachtboden, das Schachtmittelteil, der Schachtkonus sowie die Leitungen lassen sich beispielsweise mit Hilfe des Elektroschweißmuffenverfahrens oder der Stumpfschweißung miteinander verbinden.

Neben einer Gasundurchlässigkeit, die durch das Schweißen herstellbar ist, wird auf diese Art und Weise auch ein sehr fester mechanischer Verbund der einzelnen Bauteile miteinander bewerkstelligt, wodurch dem Schachtbauwerk eine hohe Festigkeit verliehen wird.

Die Höhe des Schachtbauwerks kann durch eine Variation in der Auswahl und Anzahl der vorzusehenden Schachtmittelteile gewählt werden. Vorteilhafter Weise wird die Höhe so gewählt, dass die Oberkante des Schachtkonus mit dem Geländeplanum abschließt, so dass das Schachtbauwerk das Geländeplanum nicht oder nur geringfügig überragt.

Erfindungsgemäß ist vorgesehen, dass die Gaszuführleitung und / oder die Gasabführleitung durch die Wandung eines Schachtmittelteils in das Schachtbauwerk geführt sind, wobei beispielweise durch eine vorzusehende Dichtung zwischen Wandung und Leitung der Abschnitt der Einführung fluiddicht, insbesondere wasserdicht und / oder gasdicht ausgebildet ist.

Alternativ können auch die Polymermaterialen der Wandungen des Schachtmittelteils und der Leitung miteinander verschweißt sein, wodurch ebenfalls eine Gasdichtigkeit herstellbar ist.

Auf diese Weise kann vermieden werden, dass Gas aus dem Erdreich oder entlang der Leitung in das Schachtbauwerk eindringt und dort eine kritische Konzentration erreicht.

Es ist im Rahmen der Erfindung vorteilhaft vorgesehen, dass die wenigstens eine Gaszuführleitung und die wenigstens eine Gasabführleitung dem Schachtbauwerk zugeordnet sind, was realisiert sein kann, indem die Leitungen in geringem Abstand am Schachtbauwerk vorbeigeführt sind, oder indem die Leitungen in das Schachtbauwerk eingeführt sind.

Es ist im Rahmen der Erfindung weiterhin vorgesehen, dass die wenigstens eine Gaszuführleitung und die wenigstens eine Gasabführleitung miteinander verbunden sind.

Es ist dabei von besonderem Vorteil, wenn die wenigstens eine Gaszuführleitung und/oder die wenigstens eine Gasabführleitung zum Schachtbauwerk hin ein Gefälle aufweist.

Bedingt durch das Gefälle der wenigstens einen Gaszuführleitung und/oder der wenigstens einen Gasabführleitung zum Schachtbauwerk hin wird erreicht, dass sich in der Leitung bildendes und sammelndes Kondensat aus dem Biogas durch die Kondensatleitung dem Schachtbauwerk zugeleitet wird.

Es ist dann von besonderem Vorteil, wenn an der Verbindungsstelle der wenigstens einen Gaszuführleitung und der wenigstens einen Gasabführleitung der jeweils tiefste Punkt der beiden Gefälle der beiden Leitungen angeordnet ist.

In diesem Fall sammelt sich das Kondensat an der tiefsten Stelle, nämlich an dieser Verbindung. Die Verbindung kann vorteilhafter Weise als Übergangsstück ausgebildet sein, die die Gaszuführleitung und die Gasabführleitung miteinander verbindet.

In einer besonders vorteilhaften Weiterbildung der vorliegenden Erfindung kann vorgesehen sein, dass Mittel zur Aufrechterhaltung des Drucks des Gases in der Leitung vorhanden sind.

Die Mittel zur Aufrechterhaltung des Druckes des Gases in der Leitung sind dabei so gewählt, dass Kondensat abgeleitet werden kann, ohne dass dabei Gas aus der Leitung austritt.

Ein derartiges Mittel kann so ausgelegt sein, dass es ermöglicht, das Kondensat aus der Leitung, das sich durch das Gefälle bedingt an der tiefsten Stelle der Leitung, also am Übergang von Gaszuführleitung und Gasabführleitung im Schachtbauwerk oder dem Schachtbauwerk zugeordnet sammelt, derart aus der Leitung abzuführen, dass es zu keinem Druckabfall in der Leitung kommt.

Dazu ist beispielsweise eine Abscheidearmatur installiert, die darauf beruht, dass beispielsweise eine Schwimmerkugel einen Drehschieber betätigt, der die Abscheidung des Kondensats ermöglicht.

In einer anderen technischen Lösung kann vorgesehen sein, den Abscheider vertieft anzuordnen, sodass sich über diesem eine Wassersäule aufbauen kann und der hydrostatische

Druck die Abscheideleistung insbesondere auch bei größeren Kondensatmengen und geringem Druck in der Gasleitung sicherstellt.

Eine andere technische Lösung kann durch einen Syphon oder eine ähnliche Ausformung einer Absperrung durch eine Sperrflüssigkeit geschaffen werden, bei dem insbesondere bei geringem Gasdruck beispielsweise eine Wassersäule das Gas von der Atmosphäre absperrt.

An der Verbindungsstelle von Gaszuführ- und Gasabführleitung kann ein Übergangsstück angeordnet sein, das insbesondere als T-Stück ausgebildet ist.

So kann vorgesehen sein, dass an der tiefsten Stelle des Übergangsstücks ein nach unten weisendes Rohrstück angeordnet ist, durch den das Kondensat aus der Rohrleitung austreten kann.

Es ist von großem Vorteil, wenn zu Reinigungs- und Inspektionszwecken das Übergangsstück beispielsweise durch Vorsehen von entsprechenden Flanschen von der Gaszuführ-und Gasabführleitung entfernt werden kann.

Hierbei kann in besonders einfacher Weise sowohl das Übergangsstück wie auch die Gaszuführ- und die Gasabführleitung kontrolliert, gewartet und gereinigt werden.

Es kann weiterhin zur einfachen Kontrolle, Wartung, Reinigung und Reparatur eine Reinigungs- / bzw. Inspektionsöffnung, die durch einen Blindflansch verschlossen ist, vorgesehen sein. Diese ist vorteilhaft gegenüber dem nach unten weisenden Rohrstück oder in dessen unmittelbarer Nähe angeordnet und erleichtert die vorstehend beschriebenen Arbeiten.

Es ist weiterhin erfindungsgemäß vorgesehen, dass im Schachtbauwerk eine Pumpe angeordnet ist, die dazu dient, das im Schachtbauwerk gesammelte Kondensat auszutragen und beispielsweise in einem Sammeltank oder in eine Abführleitung zu fördern.

Hierbei kann insbesondere auch vorgesehen sein, die Funktion der Pumpe mittels einer Steuerung in bestimmten Intervallen zu aktivieren.

Dies kann beispielsweise auch so geschehen, dass je nach Flüssigkeitspegel des Kondensats im Schachtbauwerk die Pumpe gestartet wird, um damit den Flüssigkeitspegel entsprechend unterhalb eines vorgegebenen Sollwertes zu halten.

Die Steuerung kann weiterhin so ausgelegt sein, dass bei einem zu hohen Kondensatspiegel im Schacht - wenn beispielsweise die Pumpe ausgefallen ist - ein entsprechendes Alarmsignal ausgegeben wird.

Im Rahmen der vorliegenden Erfindung ist es auch möglich, dass andere Mittel vorhanden sind, das Kondensat aus dem Schachtbauwerk abzuführen.

Andere Mittel können in diesem Zusammenhang beispielsweise ein Saugheber oder ein schwerkraftbasierter Abfluss oder eine vergleichbare Einrichtung sein.

Im Rahmen der vorliegenden Erfindung ist es weiterhin günstig, wenn ein Mittel zur Belüftung des Schachtbauwerks installiert ist.

Da das Schachtbauwerk in regelmäßigen Zeiträumen kontrolliert, gewartet und gereinigt werden soll, ist es günstig, wenn ein Mittel zur Belüftung des Schachbauwerks installiert ist. Als Mittel zur Belüftung des Schachtbauwerks bietet sich in diesem Zusammenhang eine Zwangsbelüftung an. Diese besteht beispielsweise aus einem Belüftungsrohr, in dem ein Belüftungsgebläse eingebaut ist.

Mit Hilfe des Belüftungsrohres, das mit dem Schachtbauwerk in Verbindung steht, ist es möglich, innerhalb einer relativ kurzen Zeit in dem Schachtbauwerk für Frischluft zu sorgen. Mit Hilfe des Belüftungsrohres kann aus der Atmosphäre Luft angesaugt werden, wobei dies mit einem Gebläse bzw. Ventilator erfolgt, die in das Schachtbauwerk eingeleitet wird. Personal, welches das Schachtbauwerk betritt, wird auf diese Weise keiner dort möglicherweise vorhandenen giftigen oder beeinträchtigenden Atmosphäre ausgesetzt. Inspektion, Wartung und Reinigung des Schachtbauwerks werden auf diese Art und Weise vereinfacht und sind insbesondere sicher durchführbar.

Es ist weiterhin von großem Vorteil, wenn im Rahmen der Erfindung vorgesehen ist, Mittel zur Übermittlung von Daten dem Schachtbauwerk zuzuordnen, um Daten mit der Vorrichtung zum Abscheiden und Auffangen von Kondensat auszutauschen.

Auf diese Weise ist es möglich, beispielsweise Art und Menge des anfallenden Kondensats in beliebig zu wählenden Zeiträumen zu übermitteln.

Es kann auch vorgesehen sein, beispielsweise auch die Funktion und den Betrieb des Abscheidesystems und der Pumpe sowie des Gebläses für die Belüftung des Schachtbauwerks zu übermitteln.

Es ist damit möglich, eine vollständige Dokumentation des Betriebs der Vorrichtung zum Abscheiden und Auffangen von Kondensat in einfacher Weise vorzunehmen.

Dies erhöht die Sicherheit der Einrichtung und ermöglicht die sinnvolle Ergänzung der Daten, die beim Betrieb beispielsweise einer Biogasanlage inklusive des zugehörigen Verstromungsaggregats gewonnen und gespeichert werden.

Im Schachtbauwerk kann eine Steighilfe angeordnet sein, beispielsweise in Form einer Leiter, die an der Innenwand des Schachtbauwerks befestigt ist. Mit Hilfe der Leiter ist das Schachtbauwerk leicht zugänglich für Personal, um Kontroll-, Wartungs- und Reinigungstätigkeiten durchzuführen.

Die Erfindung hat es sich auch zur Aufgabe gemacht, eine Gasgewinnungsvorrichtung anzugeben, das die oben beschriebenen Nachteile des Standes der Technik überwindet.

Die Erfindung betrifft damit auch eine Gasgewinnungsvorrichtung nach Anspruch 10 mit einem Schachtbauwerk mit einer Vorrichtung zum Abscheiden und Auffangen von Kondensat eines Gases.

### Kurzbeschreibung der Zeichnungen

Nachfolgend wird ein Ausführungsbeispiel der Erfindung anhand der beigefügten Zeichnungen näher erläutert.

Es zeigt:
- Fig. 1: eine geschnittene Darstellung eines erfindungsgemäßen Schachtbauwerks als Längsschnitt,
- Fig. 2: eine geschnittene Darstellung eines erfindungsgemäßen Schachtbauwerks, als Querschnitt.

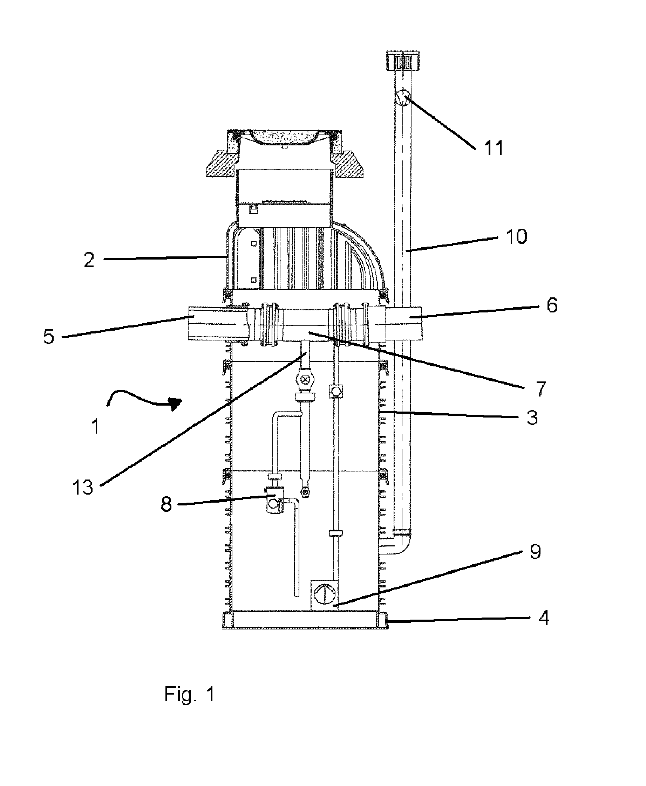

Die Fig. 1 zeigt eine geschnittene Darstellung eines erfindungsgemäßen Schachtbauwerks 1 als Längsschnitt eines Ausführungsbeispiels der Erfindung.

Das Schachtbauwerk 1 wird durch Anordnung eines Schachtbodens 4, wenigstens eines daraufgesetzten Schachtmittelteils 3 und eines auf das Schachtmittelteil 3 gesetzten Schachtkonus 2 gebildet.

Es können auch mehrere Schachtmittelteile 3 beispielsweise auch in unterschiedlicher Höhe aufeinander gesetzt angeordnet sein.

In das Schachtbauwerk 1 mündet eine Gaszuführleitung 5. Eine Gasabführleitung 6 mündet ebenfalls in das Schachtbauwerk 1. Die Leitungen 5, 6 sind durch die Wandung des Schachtmittelteils 3 geführt. Die Gaszuführleitung 5 leitet Gas von der hier nicht gezeigten Gasgewinnungsvorrichtung zur Gasabführleitung 6, die das Gas zu einem Verbraucher oder einem Speicher - die hier ebenfalls nicht gezeigt sind - weiterleitet.

Gaszuführleitung 5 und Gasabführleitung 6 sind durch ein Übergangsstück 7 miteinander gasdicht verbunden. Da die Gaszuführleitung 5 und die Gasabführleitung 6 zum Schachtbauwerk 1 hin ein Gefälle aufweisen, ist am tiefsten Punkt des Übergangsstücks 7 ein Rohrstutzen angebracht, der über eine Kondensatleitung (13) in einen Kondensatabscheider 8 mündet.

Im Schachtbauwerk 1 ist weiterhin eine Pumpe 9 angeordnet, die abgeschiedenes Kondensat aus dem Schachtbauwerk 1 hinausfördert.

Dem Schachtbauwerk 1 sind weiterhin Mittel zur Belüftung 10 zugeordnet, dies kann beispielsweise ein Belüftungsrohr sein.
Dem Mittel zur Belüftung 10 ist ein Ventilator 11 zugeordnet.

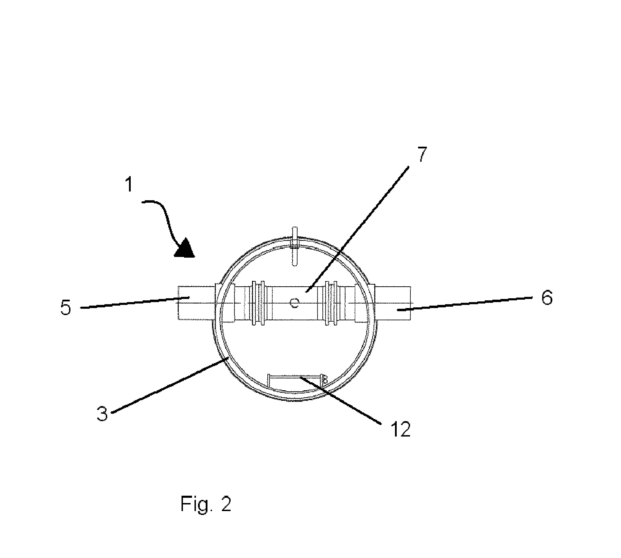

Figur 2 zeigt ein erfindungsgemäßes Schachtbauwerk 1 in einem Querschnitt.

Im Schachtbauwerks 1 münden sowohl eine Gaszuführleitung 5 wie auch eine Gasabführleitung, die durch die Wandung des Schachtmittelteils 3 geführt sind.

Gaszuführleitung 5 und Gasabführleitung 6 sind durch ein Übergangsstück 7 miteinander verbunden.

Im Schachtbauwerk 1 ist weiterhin eine Steighilfe 12, beispielsweise in Form einer an der Innenwand des Schachtbauwerks 1 befestigten Leiter angeordnet.

### Bezugszeichenliste

- 1: Schachtbauwerk
- 2: Schachtkonus
- 3: Schachtmittelteil
- 4: Schachtboden
- 5: Gaszuführleitung
- 6: Gasabführleitung
- 7: Übergangsstück
- 8: Kondensatabscheider
- 9: Pumpe
- 10: Mittel zur Belüftung
- 11: Ventilator
- 12: Steighilfe
- 13: Kondensatleitung

## Patentansprüche

1. Schachtbauwerk (1) mit einer Einrichtung zur Abtrennung von Kondensaten eines Gases, insbesondere eines Biogases, mit einem Schachtboden (4), optional wenigstens einem Schachtmittelteil (3), optional einem Schachtkonus (2), wenigstens einer zugeordneten Gaszuführleitung (5), wenigstens einer zugeordneten Gasabführleitung (6), wenigstens einer Kondensatleitung (13) und wenigstens einer Vorrichtung zum Abscheiden und Auffangen von Kondensat aus Gaszuführ- und / oder Gasabführleitung (5, 6).

2. Schachtbauwerk (1) nach Anspruch 1, **dadurch gekennzeichnet, dass** der Schachtboden (4), das Schachtmittelteil (3), der Schachtkonus (2), die wenigstens eine Gaszuführleitung (5), die wenigstens eine Gasabführleitung (6), die wenigstens eine Kondensatleitung (13) und die wenigstens eine Vorrichtung zum Abscheiden und Auffangen von Kondensat wenigstens anteilig aus Polymermaterial bestehen.

3. Schachtbauwerk (1) nach Anspruch 1 oder 2, **dadurch gekennzeichnet, dass** die wenigstens eine Gaszuführleitung (5) und die wenigstens eine Gasabführleitung (6) miteinander verbunden sind.

4. Schachtbauwerk (1) nach einem der vorangehenden Ansprüche, **dadurch gekennzeichnet, dass** die wenigstens eine Gaszuführleitung (5) und / oder die wenigstens eine Gasabführleitung (6) zum Schachtbauwerk (1) hin ein Gefälle aufweist.

5. Schachtbauwerk (1) nach einem der vorangehenden Ansprüche, **dadurch gekennzeichnet, dass** Mittel zur Aufrechterhaltung des Drucks des Gases in der Leitung (5, 6) vorhanden sind.

6. Schachtbauwerk (1) nach einem der vorangehenden Ansprüche, **dadurch gekennzeichnet, dass** die Einführung der wenigstens einen Gaszuführleitung (5) und / oder der wenigstens einen Gasabführleitung (6) in das Schachtbauwerk (1) fluiddicht und / oder gasdicht ausgebildet ist.

7. Schachtbauwerk (1) nach einem der vorangehenden Ansprüche, **dadurch gekennzeichnet, dass** ein Mittel vorhanden ist, das Kondensat aus dem Schachtbauwerk (1) abzuführen.

8. Schachtbauwerk (1) nach einem der vorangehenden Ansprüche, **dadurch gekennzeichnet, dass** ein Mittel zur Belüftung des Schachtbauwerks (1) installiert ist.

9. Schachtbauwerk (1) nach einem der vorangehenden Ansprüche, **dadurch gekennzeichnet, dass** Mittel zur Übermittlung von Daten vorhanden sind, um Daten mit der Vorrichtung zum Abscheiden und Auffangen von Kondensat auszutauschen.

10. Gasgewinnungsvorrichtung mit einem Schachtbauwerk (1) mit einer Vorrichtung zum Abscheiden und Auffangen von Kondensat eines Gases nach einem der Ansprüche 1 bis 9.
